# EUROPEAN PATENT SPECIFICATION

(11) **EP 1 543 135 B1**
(45) Date of publication and mention of the grant of the patent: **17.12.2008**
(21) Application number: 03750589.8
(22) Date of filing: 22.09.2003
(51) Int. Cl.: C12P 17/04, C12P 7/58

(54) **PROCESS FOR PRODUCING L-ASCORBIC ACID**
VERFAHREN ZUR HERSTELLUNG VON L-ASCORBINSÄURE
PROCEDE POUR PRODUIRE DE L'ACIDE L-ASCORBIQUE

(30) Priority: 27.09.2002 EP 02021602
(43) Date of publication of application: 22.06.2005
(73) Proprietor: DSM IP Assets B.V., 6411 TE Heerlen (NL)
(72) Inventor: HOSHINO, Tatsuo, Kamakura-shi, Kanagawa-ken 248-0027 (JP); SHINJOH, Masako, Ch-4054 Basel (CH)
(74) Representative: Schwander, Kuno
(86) International application number: PCT/EP2003/010489
(87) International publication number: WO 2004/029267

(56) References cited:
- EP-A- 0 832 974
- EP-A- 0 911 415
- WO-A-20/04029268
- US-B1- 6 242 233
- SAITO Y ET AL: "CLONING OF GENES CODING FOR L-SORBOSE AND L-SORBOSONE DEHYDROGENASES FROM GLUCONOBACTER OXYDANS AND MICROBIAL PRODUCTION OF 2-KETO-L-GULONATE, A PRECURSOR OF L-ASCORBIC ACID, IN A RECOMBINANT G. OXYDANS STRAIN" APPLIED AND ENVIRONMENTAL MICROBIOLOGY, WASHINGTON,DC, US, vol. 63, no. 2, 1997, pages 454-460, XP000886144 ISSN: 0099-2240
- SUGISAWA T ET AL: "ISOLATION AND CHARACTERIZATION OF A NEW VITAMIN C PRODUCING ENZYME (L-GULONO-GAMMA-LACTONE DEHYDROGENASE) OF BACTERIAL ORIGIN" BIOSCIENCE, BIOTECHNOLOGY AND BIOCHEMISTRY, vol. 59, no. 2, February 1995 (1995-02), pages 190-196, XP001084987 ISSN: 0916-8451
- MACAULEY S ET AL: "THE GENUS GLUCONOBACTER AND ITS APPLICATIONS IN BIOTECHNOLOGY" CRC CRITICAL REVIEWS IN BIOTECHNOLOGY, CRC PRESS, BOCA RATON, FL, US, vol. 21, no. 1, 2001, pages 1-25, XP008026977 ISSN: 0738-8551
- HANCOCK R D ET AL: "Biotechnological approaches for l-ascorbic acid production" TRENDS IN BIOTECHNOLOGY, ELSEVIER PUBLICATIONS, CAMBRIDGE, GB, vol. 20, no. 7, 1 July 2002 (2002-07-01), pages 299-305, XP004361398 ISSN: 0167-7799
- BOUDRANT J: "MICROBIAL PROCESSES FOR ASCORBIC ACID BIOSYNTHESIS: A REVIEW" ENZYME AND MICROBIAL TECHNOLOGY, STONEHAM, MA, US, vol. 12, no. 5, 1 May 1990 (1990-05-01), pages 322-329, XP000646789 ISSN: 0141-0229

## Description

The present invention relates to the use of Enzyme B of *Gluconobacter oxydans* DSM 4025 as disclosed in EP 0 832 974 A2 in a process for producing L-ascorbic acid.

Feasibility studies on the biotechnological synthesis of L-ascorbic acid (vitamin C) were performed for many years since the "Reichstein method" was established in 1934. The microorganisms *Gluconobacter oxydans* DSM 4025 or *Escherichia coli* carrying the D-arabinono-1,4-lactone oxidase gene of *Saccharomyces cerevisiae*, *Candida albicans* and *Saccharomyces cerevisiae* oxidize L-galactono-1,4-lactone to vitamin C. *Saccharomyces cerevisiae* and *Candida albicans* possess a D-arabinose dehydrogenase catalyzing the production of D-arabinono-1,4-lactone and L-galactono-1,4-lactone from D-arabinose and L-galactose, respectively. However, there were no reports describing the possibility of biological vitamin C production from another L-hexose as intermediate, that is, L-idose, L-gulose, and L-talose, with a configuration corresponding to that of vitamin C (C4 and C5 positions).

The present invention provides the use of Enzyme B of *G*. *oxydans* DSM 4025, as disclosed in EP 0 832 974 A2, in a process for producing L-ascorbic acid from L-gulose, L-galactose, L-gulono-1,4-lactone (and its acid form, L-gulonic acid) and from L-galactono-1,4-lactone (and its acid form, L-galactonic acid).

The present invention also provides the use of Enzyme B of *G. oxydans* DSM 4025, as disclosed in EP 0 832 974 A2, in a process for producing L-gulono-1,4-lactone or L-galactono-1,4-lactone, or their acid forms L-gulonic acid or L-galactonic acid from L-gulose or L-galactose, respectively.

EP 0 832 974 A2 discloses Enzyme B *of G. oxydans* DSM 4025 having the following physico-chemical properties:
(a) molecular weight of about 60,000 Da on SDS-PAGE;
(b) substrate specificity for primary and secondary alcohols and aldehydes;
(c) pH-stability at pH of about 6 to about 9;
(d) pH-optimum at pH of about 8.0; and
(e) inhibited by Cu²⁺, Zn²⁺, Mn²⁺, Fe²⁺, and Fe³⁺.

The present invention is related to a process for the production of L-ascorbic acid with an enzyme having the amino acid sequence of SEQ ID NO: 2 or an amino acid sequence that is 90% identical thereto, with the activity to produce L-ascorbic acid, whereby L-ascorbic acid is produced from a substrate which is selected from the group consisting of L-gulose and L-galactose.

L-Hexoses like L-gulose and L-galactose are rare sugars which are basically produced by chemical methods and are commercially high-cost compounds. However, biological preparations for L-gulose and L-galactose have been recently reported. L-Gulose production from D-sorbitol by Enzyme A of *G*. *oxydans* DSM 4025 was reported in EP 0 832 974 A2. L-Gulose production from L-sorbose by L-ribose isomerase was disclosed in US 6,037,153. L-Galactose production from L-sorbose is reported by Izumori et al. (2001 Annual Meeting of the Society for Bioscience and Bioengineering, Japan). In this process they combined two enzymatic processes consisting of "L-sorbose to L-tagatose" reaction with L-tagatose epimerase of *Pseudomonas cichorii* ST-24 strain (US 5,811,271) and "L-tagatose to L-galactose" reaction with D-arabinose isomerase of *Bacillus stearothermophilus* 14a strain. L-Gulono-1,4-lactone may be prepared from D-glucose.

In another aspect the present invention provides a process for
(a) producing
   (i) L-ascorbic acid from L-gulose or L-galactose by an enzyme which comprises contacting L-gulose or L-galactose with an enzyme having an amino acid sequence of SEQ ID NO: 2 or an amino acid sequence that is 90% identical thereto, or a portion thereof, with the activity to produce L-ascorbic acid from L-gulose and L-galactose or a functional equivalent thereof, in a reaction mixture,
   (ii) L-gulono-1,4-lactone from L-gulose, or L-galactono-1,4-lactone from L-galactose by an enzyme which comprises contacting L-gulose or L-galactose with an enzyme having an amino acid sequence of SEQ ID NO:2 or an amino acid sequence that is 90% identical thereto, or a portion thereof, with the activity to produce L-gulono-1,4-lactone from L-gulose and L-galactono-1,4-lactone from L-galactose, or a functional equivalent thereof, in a reaction mixture, or
   (iii) L-ascorbic acid from L-gulono-1,4-lactone or from L-galactono-1,4-lactone by an enzyme which comprises contacting L-gulono-1,4-lactone or from L-galactono-1,4-lactone with the enzyme having an amino acid sequence of SEQ IDNO: 2 or an amino acid sequence that is 90% identical thereto, or a portion thereof, with the activity to produce L-ascorbic acid from L-gulono-1,4-lactone and from L-galactono-1,4-lactone, or a functional equivalent thereof, in a reaction mixture,
      and
(b) isolating L-ascorbic acid, L-gulono-1,4-lactone or L-galactono-1,4-lactone from the reaction mixture.

It is one embodiment of the present invention to provide a process for the production of L-ascorbic acid with an enzyme having the amino acid sequence of SEQ IDNO: 2 or an amino acid sequence that is 90% identical thereto, with the activity to produce L-ascorbic acid, whereby L-ascorbic acid is produced from a substrate which is selected from the group consisting of L-gulono-1,4-lactone, L-gulonic acid, L-galactono-1,4-lactone and L-galactonic acid.

In a further embodiment, the present invention covers a process for the production of L-gulono-1,4-lactone or L-gulonic acid with an enzyme having the amino acid sequence of SEQ ID NO: 2 or an amino acid sequence that is 90% identical thereto, with the activity to produce L-gulono-1,4-lactone or L-gulonic acid, whereby L-gulono-1,4-lactone or L-gulonic acid is produced from L-gulose.

Furthermore, it is an aspect of the present invention to provide a process for the production of L-galactono-1,4-lactone or L-galactonic acid with an enzyme having the amino acid sequence of SEQ ID NO: 2 or an amino acid sequence that is 90% identical thereto, with the activity to produce L-galactono-1,4-lactone or L-galactonic acid, whereby L-galactono-1,4-lactone or L-galactonic acid is produced from L-galactose.

A use of enzyme B in a process according to the present invention as described above comprises (a) contacting the enzyme with the respective substrate and (b) isolating the respective product from the reaction mixture. In case of vitamin C as product, the substrate is selected from the group consisting of L-gulose, L-galactose, L-gulono-1,4-lactone, L-gulonic acid, L-galactono-1,4-lactone and L-galactonic acid. In case of L-gulono-1,4-lactone or L-gulonic acid as product, the substrate is L-gulose. In case of L-galactono-1,4-lactone or L-galactonic acid as product, the substrate is L-galactose.

In the present invention, a functional equivalent of the enzyme can be made either by chemical peptide synthesis known in the art or by recombinant means on the basis of the DNA sequences as disclosed herein by methods known in the state of the art. Amino acid exchanges in proteins and peptides which do not generally alter the activity of such molecules are known in the state of the art. The most commonly occurring exchanges are: Ala/Ser, Val/Ile, Asp/Glu, Thr/Ser, Ala/Gly, Ala/Thr, Ser/Asn, Ala/Val, Ser/Gly, Tyr/Phe, Ala/Pro, Lys/Arg, Asp/Asn, Leu/Ile, Leu/Val, Ala/Glu, Asp/Gly as well as these in reverse.

Furthermore such functional equivalent of the enzyme includes an amino acid sequence encoded by a DNA sequence of SEQ ID NO: 1 as disclosed, e.g., in the sequence listing as well as the complementary strand, or those which include the sequences, DNA sequences which hybridize under stringent hybridization and washing conditions with such sequences or fragments thereof and DNA sequences, which because of the degeneration of the genetic code, do not hybridize under stringent hybridization and washing conditions with such sequences but which code for polypeptides having exactly the same amino acid sequence, wherein the functional equivalent has the enzymatic activity of producing
(i) L-ascorbic acid from L-gulose and from L-galactose,
(ii) L-gulono-1,4-lactone (and its acid form, L-gulonic acid) from L-gulose and L-galactono-1,4-lactone (and its acid form, L-galactonic acid) from L-galactose, or
(iii) L-ascorbic acid from L-gulono-1,4-lactone (and its acid form, L-gulonic acid) and from L-galactono-1,4-lactone (and its acid form, L-galactonic acid).

"Standard conditions" for hybridization mean in this context the conditions which are generally used by a man skilled in the art to detect specific hybridization signals, or preferably so called stringent hybridization and non-stringent washing conditions or more preferably so called stringent hybridization and stringent washing conditions a man skilled in the art is familiar with. Furthermore, DNA sequences which can be made by the polymerase chain reaction by using primers designed on the basis of the DNA sequences disclosed herein by methods known in the art are also an object of the present invention. It is understood that the DNA sequences of the present invention can also be made synthetically as described, e.g. in EP 747 483 A2.

A mutant of the gene can be prepared by treating the gene or a microorganism carrying the gene with a mutagen such as ultraviolet irradiation, X-ray irradiation, γ-ray irradiation or contact with a nitrous acid, N-methyl-N'-nitro-N-nitrosoguanidine (NTG), or other suitable mutagens, or isolating a colony or clone occurring by spontaneous mutation or by standard methods of *in vitro* mutagenesis known in the art. Many of these methods have been described in various publications.

As used herein, a "mutant" is any gene that encodes a non-native polynucleotide sequence or a polynucleotide sequence that has been altered from its native form (such as, e.g., by rearrangement or deletion or substitution of from 1-100, preferably 20-50, more preferably less than 10 nucleotides). As noted above, such a non-native sequence may be obtained by random mutagenesis, chemical mutagenesis, spontaneous mutation, UV-irradiation, PCR-prone error generation, site-directed mutagenesis, and the like. Preferably, the mutation results in the expression of a polypeptide having the increased production or improved activity compared to a non-mutant parental polypeptide using the assay procedures set forth in the Examples. Methods for generating, screening for, and identifying such mutant cells are well known in the art.

A specific and preferred *G*. *oxydans* strain as a donor of a DNA sequence encoding a polypeptide has been deposited at the Deutsche Sammlung von Mikroorganismen und Zellkulturen (DSMZ) in Göttingen (Germany) under DSM No. 4025, which was done under the stipulations of the Budapest Treaty on March 17,1987. A biologically and/or taxonomically homogeneous culture of a microorganism having the identifying characteristics *G*. *oxydans* DSM 4025 can also be a donor of the DNA sequence.

A subculture of *G*. *oxydans* DSM 4025 has been deposited in the Agency of Industrial Science and Technology, Fermentation Research Institute, Japan, under the deposit No.: FERM BP-3812. EP 0 278 447 B1 discloses the characteristics of this strain.

By using the information of the so determined nucleotide sequence (in consideration of the codon usage) a gene encoding evolutionally divergent enzyme having the activity of producing (i) L-ascorbic acid from L-gulose and L-galactose, (ii) L-gulono-1,4-lactone (and its acid form, L-gulonic acid) from L-gulose and L-galactono-1,4-lactone (and its acid form, L-galactonic acid) from L-galactose, or (iii) L-ascorbic acid from L-gulono-1,4-lactone (and its acid form, L-gulonic acid) and from L-galactono-1,4-lactone (and its acid form, L-galactonic acid), vitamin C forming-activity from L-gulose or L-galactose, can be isolated from a different organism by colony- or Southern-hybridization with a probe synthesized according to the amino acid sequence deduced from said nucleotide sequence or by the polymerase chain reaction with primers also synthesized according to said information, if necessary.

Furthermore, a preferred host microorganism for constructing a recombinant microorganism carrying Enzyme B gene of *G*. *oxydans* DSM 4025 and its functional equivalent or mutant defined above may be *Escherichia coli*, *Pseudomonas putida* and *G. oxydans* DSM 4025 and their biologically and/or taxonomically homogeneous culture or mutant.

To construct a recombinant microorganism carrying the gene for Enzyme B and its functional equivalent or mutant on a recombinant expression vector or on a chromosomal DNA of a host microorganism, various gene transfer methods including transformation, transduction, conjugal mating, and electroporation can be used. The method for constructing a recombinant organism may be selected from the methods well known in the field of molecular biology. Usual transformation systems can be used for *Escherichia coli,* and *Pseudomonas*. A transduction system can also be used for *Escherichia coli*. Conjugal mating systems can be widely used in Gram-positive and Gram-negative bacteria including *E*. *coli*, *Pseudomonas putida* and *G*. *oxydans*. The conjugation can occur in liquid media or on a solid surface. The preferred recipient is selected from *E*. *coli, Pseudomonas putida* and *G*. *oxydans* which can produce active Enzyme B with a suitable recombinant expression vector. To the recipient for conjugal mating, a selective marker is usually added; for example, resistance against nalidixic acid or rifampicin is usually selected.

The microorganisms provided in the present invention may be cultured in an aqueous medium supplemented with appropriate nutrients under aerobic conditions. The cultivation may be conducted at a pH between about 1.0 and about 9.0, preferably between about 2.0 and about 8.0. While the cultivation period varies depending upon pH, temperature and nutrient medium used, usually 2 to 5 days will bring about favorable results. A suitable temperature range for carrying out the cultivation is from about 13°C to about 45°C, preferably from about 18°C to about 42°C.

Thus, the process for the production of vitamin C of the present invention as described above is conducted for 1 to 120 h at a pH of about 1 to about 9 and at a temperature of about 13°C to about 45°C. Preferably, the process is conducted at a pH of about 2 to about 8 and at a temperature of about 13°C to about 45°C. These conditions are also applied to the process for the production of L-gulono-1,4-lactone, L-gulonic acid, L-galactono-1,4-lactone or L-galactonic acid, as further embodiments of the present invention.

It is usually required that the culture medium contains such nutrients as assimilable carbon sources, digestible nitrogen sources and inorganic substances, vitamins, trace elements and other growth promoting factors. Examples for assimilable carbon sources include glycerol, D-glucose, D-mannitol, D-fructose, D-arabitol, D-sorbitol and L-sorbose.

Various organic or inorganic substances may also be used as nitrogen sources, such as yeast extract, meat extract, peptone, casein, corn steep liquor, urea, amino acids, nitrates, ammonium salts and the like. As inorganic substances, magnesium sulfate, potassium phosphate, ferrous and ferric chlorides, calcium carbonate and the like may be used.

As used herein, L-ascorbic acid means that the substance exists either as a free acid form or as a salt form such as Na-salt, K-salt, or hemicalcium-salt, such as calcium L-ascorbate dihydrate. Moreover, concentration of L-ascorbic acid is described as the free acid form unless otherwise stated.

As used herein, L-gulono-1,4-lactone and L-galactono-1,4-lactone mean that the substances exist as their lactone forms and/or their acid forms, both of which exist in an equilibrium state under various physico-chemical conditions.

As used herein the phrase "standard conditions for hybridization" means conditions which are generally used by a person skilled in the art to detect specific hybridization signals, or preferably, so called stringent hybridization and non-stringent washing conditions, or more preferably, so called moderately stringent conditions, or even more preferably, so called stringent hybridization and stringent washing conditions which a person skilled in the art is familiar with.

For example, any combination of the following hybridization and wash conditions may be used, as appropriate:
*High Stringency Hybridization*: 6X SSC, 0.5% SDS,100 µg/ml denatured salmon sperm DNA, 50% formamide, incubate overnight at 42°C with gentle rocking.
*High Stringency Wash*: 1 wash in 2X SSC, 0.5% SDS at room temperature for 15 minutes, followed by another wash in 0.1X SSC, 0.5% SDS at room temperature for 15 minutes.
*Low Stringency Hybridization*: 6X SSC, 0.5% SDS, 100 µg/ml denatured salmon sperm DNA, 50% formamide, incubate overnight at 37°C with gentle rocking.
*Low Stringency Wash*: 1 wash in 0.1X SSC, 0.5% SDS at room temperature for 15 minutes.

Moderately stringent conditions may be obtained by varying the temperature at which the hybridization reaction occurs and/or the wash conditions as set forth above.

The concentration of the substrates including L-aldose and L-aldonolactone in a reaction mixture can vary depending on other reaction conditions, but, in general, may be between about 1 g/l and about 300 g/l, preferably between about 10 g/l and about 200g/l.

The reaction can be conducted aerobically.

For the reaction, the enzyme can be used in any form, including, but not limited to, enzyme solution, immobilized enzyme, intact cells, and immobilized cells may be used.

After the reaction, L-ascorbic acid or L-aldonolactone may be recovered from the reaction mixture by the combination of various kinds of chromatography, for example, thin layer chromatography, adsorption chromatography, ion-exchange chromatography, gel filtration chromatography or high performance liquid chromatography. L-Aldonolactone as a reaction product can also be used as a substrate for a further reaction as it is in the reaction mixture of this invention without purification.

The following examples are provided to further illustrate the process of the present invention. These examples are illustrative only and are not intended to limit the scope of the invention in any way.

### Example 1: Production of L-gulono-1,4-lactone/L-gulonic acid from L-gulose by Escherichia coli JM109 carrying the Enzyme B gene

The gene encoding Enzyme B was cloned and subcloned from pSS103R into a vector pTrcMalE to construct pTrcMalE-EnzB as described in EP 0 832 974 A2. *Escherichia coli* JM109 carrying pTrcMalE-EnzB was grown on 2 ml of LB medium with 100 µg/ml of ampicillin at 30°C for 15 h and 100 µl of the resulting broth was transferred into fresh LB medium with 100 µg/ml of ampicillin and incubated at 30°C for 4 h. Then, IPTG was added to the culture at a final concentration of 0.2 mM and the culture was further cultivated at 30°C for 2 h. The control cultivation of *E*. *coli* JM109 carrying pTrcMalE-EnzB without IPTG addition was also performed. *Escherichia coli* JM109 was used as the control strain in the same manner as described above, cultivation with or without addition of IPTG. The cells from 8 ml of the culture were collected by centrifugation and suspended in 1 ml of distilled water. The resulting cell suspension was used for the reaction with 400 µl of reaction mixture consisting of 250 µl of the cell suspension, 1% substrate, 0.3% NaCl, 1% CaCO₃, 1 µg/ml of PQQ and 1 mM PMS and the mixture was incubated at room temperature for 16 h. The substrate used in this experiment was L-gulose. The amounts of L-gulono-1,4-lactone plus L-gulonic acid and vitamin C are summarized in Table 1.

**Table 1**

| Strain | IPTG | HPLC | |
|---|---|---|---|
| | | L-GuL+L-GuA (mM) | Vitamin C (mg/L) |
| JM109 /pTrcMalE-EnzB | + | 8.4 | 42.5 |
| | - | 4.6 | 10.5 |
| JM109 | + | nd | nd |
| | - | nd | nd |

| | | | |
|---|---|---|---|
| L-GuL: L-gulono-1,4-lactone; L-GuA: L-gulonic acid; nd: not detected | | | |

### Example 2: Production of vitamin C from L-gulono-1,4-lactone/L-gulonic acid by Escherichia coli JM109 carrying the Enzyme B gene

The experiment was performed as described in Example 1, except for the substrate used which was L-gulono-1,4-lactone in this Example. The amount of vitamin C is summarized in Table 2.

**Table 2**

| Strain | IPTG | HPLC |
|---|---|---|
| | | Vitamin C (mg/L) |
| JM109/pTrcMalE-EnzB | + | 1.4 |
| | - | 1.2 |
| JM109 | + | nd |
| | - | nd |

| | | |
|---|---|---|
| nd: not detected | | |

### Example 3: Production of L-galactono-1,4-lactone/L-galactonic acid from L-galactose by Escherichia coli JM109 carrying the Enzyme B gene

The experiment was performed as described in Example 1, except for the substrate used which was L-galactose in this Example. The amounts of L-galactono-1,4-lactone plus L-galactonic acid and vitamin C are summarized in Table 3.

**Table 3**

| Strain | IPTG | HPLC | |
|---|---|---|---|
| | | L-GaL+L-GaA (mM) | Vitamin C (mg/L) |
| JM109/pTrcMalE-EnzB | + | 6.2 | 2.7 |
| | - | 3.9 | 1.6 |
| JM109 | + | nd | nd |
| | - | nd | nd |

| | | | |
|---|---|---|---|
| L-GaL: L-galactono-1,4-lactone; L-GaA: L-galactonic acid; nd: not detected | | | |

### Example 4: Production of vitamin C from L-galactono-1,4-lactone/L-galactonic acid by Escherichia coli JM109 carrying the Enzyme B gene

The experiment was performed as described in Example 1, except for the substrate used which was L-galactono-1,4-lactone in this Example. The amount of vitamin C is summarized in Table 4.

**Table 4**

| Strain | IPTG | HPLC |
|---|---|---|
| | | Vitamin C (mg/L) |
| JM109/pTrcMalE-EnzB | + | 4.7 |
| | - | 3.7 |
| JM109 | + | nd |
| | - | nd |

| | | |
|---|---|---|
| nd: not detected | | |

## Claims

1. Use of enzyme B obtainable from *GLUCONOBACTER oxydans* DSM 4025 for producing L-ascorbic acid by contacting said enzyme with a substrate which is selected from the group consisting of L-gulose, L-galactose, L-gulono-1,4-lactone, L-gulonic acid, L-galactono-1,4-lactone, and L-galactonic acid; wherein said enzyme is selected from the group consisting of:
(a) an amino acid sequence according to SEQ ID NO: 2,
(b) an amino acid sequence which is 90% identical to SEQ ID NO: 2 with the activity to produce L-ascorbic acid, and
(c) an amino acid sequence encoded by a DNA sequence of SEQ ID NO: 1 or a DNA sequence with hybidizes under stringent hybridization and washing conditions with said sequence with the activity to produce L-ascorbic acid.

2. Use of enzyme B obtainable from *G*. *oxydans* DSM 4025 for producing L-gulono-1,4-lactone or L-gulonic acid by contacting said enzyme with L-gulose, wherein said enzyme is selected from the group consisting of:
(a) an amino acid sequence according to SEQ ID NO: 2,
(b) an amino acid sequence which is 90% identical to SEQ ID NO: 2 with the activity to produce L-ascorbic acid, and
(c) an amino acid sequence encoded by a DNA sequence of SEQ ID NO: 1 or a DNA sequence with hybidizes under stringent hybridization and washing conditions with said sequence with the activity to produce L-ascorbic acid.

3. Use of enzyme B obtainable from *G*. *oxydans* DSM 4025 for producing L-galactono-1,4-lactone or galactonic acid by contacting said enzyme with L-galactose, wherein said enzyme is selected from the group consisting of:
(a) an amino acid sequence according to SEQ ID NO: 2,
(b) an amino acid sequence which is 90% identical to SEQ ID NO: 2 with the activity to produce L-ascorbic acid, and
(c) an amino acid sequence encoded by a DNA sequence of SEQ ID NO: 1 or a DNA sequence with hybidizes under stringent hybridization and washing conditions with said sequence with the activity to produce L-ascorbic acid.

4. Use according to any one of claims 1 to 3 comprising (a) contacting the enzyme with the respective substrate and (b) isolating the product which is selected from the group consisting ofL-ascorbic acid, L-gulono-1,4-lactone, L-gulonic acid, L-galactono-1,4-lactone, and L-galactonic acid from the reaction mixture.

5. Use according to any one of claims I to 4, wherein the process is conducted for 1 to 120 h at a pH of 1 to 9 and at a temperature of 13°C to 45°C.

6. Use according to claim 5, wherein the process is conducted at a pH of 2 to 8 and at a temperature of 18°C to 42°C.

7. A process for the production of L-ascorbic acid from a substrate selected from the group consisting of L-gulose, L-galactose, L-gulono-1,4-lactone, L-gulonic acid, L-galactono-1,4-lactone, and L-galactonic acid; said process comprising contacting enzyme B obtainable from *G*. *oxydans* DSM 4025 with said substrate and wherein said enzyme is selected from the group consisting of:
(a) an amino acid sequence according to SEQ ID NO: 2,
(b) an amino acid sequence which is 90% identical to SEQ ID NO: 2 with the activity to produce L-ascorbic acid, and
(c) an amino acid sequence encoded by a DNA sequence of SEQ ID NO: 1 or a DNA sequence with hybidizes under stringent hybridization and washing conditions with said sequence with the activity to produce L-ascorbic acid.

8. A process for the production of L-gulono-1,4-lactone or L-gulonic acid from Lgulose, said process comprising contacting enzyme B obtainable from *G*. *oxydans* DSM 4025 with L-gulose and wherein said enzyme is selected from the group consisting of:
(a) an amino acid sequence according to SEQ ID NO: 2,
(b) an amino acid sequence which is 90% identical to SEQ ID NO: 2 with the activity to produce L-ascorbic acid, and
(c) an amino acid sequence encoded by a DNA sequence of SEQ ID NO: 1 or a DNA sequence with hybidizes under stringent hybridization and washing conditions with said sequence with the activity to produce L-ascorbic acid.

9. A process for the production of L-galactono-1,4-lactone or galactonic acid from L-galactose, said process comprising contacting enzyme B obtainable from *G*. *oxydans* DSM 4025 with L-galactose and wherein said enzyme is selected from the group consisting of:
(a) an amino acid sequence according to SEQ ID NO: 2,
(b) an amino acid sequence which is 90% identical to SEQ ID NO: 2 with the activity to produce L-ascorbic acid, and
(c) an amino acid sequence encoded by a DNA sequence of SEQ ID NO: 1 or a DNA sequence with hybidizes under stringent hybridization and washing conditions with said sequence with the activity to produce L-ascorbic acid.

## Patentansprüche

1. Verwendung von aus *GLUCONOBACTER oxydans* DSM 4025 erhältlichem Enzym B zur Herstellung von L-Ascorbinsäure durch Inkontaktbringen des Enzyms mit einem Substrat, das aus der Gruppe bestehend aus L-Gulose, L-Galactose, L-Gulono-1,4-lacton, L-Gulonsäure, L-Galactono-1,4-lacton und L-Galactonsäure ausgewählt ist, wobei das Enzym ausgewählt ist aus der Gruppe, bestehend aus:
(a) einer Aminosäuresequenz gemäß SEQ ID NO: 2,
(b) einer Aminosäuresequenz mit 90% Identität zu SEQ ID NO: 2 und der Aktivität zur Produktion von L-Ascorbinsäure sowie
(c) einer von einer DNA-Sequenz der SEQ ID NO: 1 oder einer mit dieser Sequenz unter stringenten Hybridisierungs- und Waschbedingungen hybridisierenden DNA-Sequenz codierten Aminosäuresequenz mit der Aktivität zur Produktion von L-Ascorbinsäure.

2. Verwendung von aus *G*. *oxydans* DSM 4025 erhältlichem Enzym B zur Herstellung von L-Gulono-1,4-lacton oder L-Gulonsäure durch Inkontaktbringen des Enzyms mit L-Gulose, wobei das Enzym ausgewählt ist aus der Gruppe, bestehend aus:
(a) einer Aminosäuresequenz gemäß SEQ ID NO: 2,
(b) einer Aminosäuresequenz mit 90% Identität zu SEQ ID NO: 2 und der Aktivität zur Produktion von L-Ascorbinsäure sowie
(c) einer von einer DNA-Sequenz der SEQ ID NO: 1 oder einer mit dieser Sequenz unter stringenten Hybridisierungs- und Waschbedingungen hybridisierenden DNA-Sequenz codierten Aminosäuresequenz mit der Aktivität zur Produktion von L-Ascorbinsäure.

3. Verwendung von aus *G*. *oxydans* DSM 4025 erhältlichem Enzym B zur Herstellung von L-Galactono-1,4-lacton oder L-Galactonsäure durch Inkontaktbringen des Enzyms mit L-Galactose, wobei das Enzym ausgewählt ist aus der Gruppe, bestehend aus:
(a) einer Aminosäuresequenz gemäß SEQ ID NO: 2,
(b) einer Aminosäuresequenz mit 90% Identität zu SEQ ID NO: 2 und der Aktivität zur Produktion von L-Ascorbinsäure sowie
(c) einer von einer DNA-Sequenz der SEQ ID NO: 1 oder einer mit dieser Sequenz unter stringenten Hybridisierungs- und Waschbedingungen hybridisierenden DNA-Sequenz codierten Aminosäuresequenz mit der Aktivität zur Produktion von L-Ascorbinsäure.

4. Verwendung nach einem der Ansprüche 1 bis 3, umfassend (a) das Inkontaktbringen des Enzyms mit dem entsprechenden Substrat und (b) das Isolieren des aus der Gruppe bestehend aus L-Ascorbinsäure, L-Gulono-1,4-lacton, L-Gulonsäure, L-Galactono-1,4-lacton und L-Galactonsäure ausgewählten Produkts aus dem Reaktionsgemisch.

5. Verwendung nach einem der Ansprüche 1 bis 4, wobei das Verfahren 1 bis 120 h bei einem pH-Wert von 1 bis 9 und einer Temperatur von 13 °C bis 45 °C ausgeführt wird.

6. Verwendung nach Anspruch 5, wobei das Verfahren bei einem pH-Wert von 2 bis 8 und einer Temperatur von 18 °C bis 42 °C ausgeführt wird.

7. Verfahren zur Herstellung von L-Ascorbinsäure aus einem aus der Gruppe bestehend aus L-Gulose, L-Galactose, L-Gulono-1,4-lacton, L-Gulonsäure, L-Galactono-1,4-lacton und L-Galactonsäure ausgewählten Substrat ist, wobei man in dem Verfahren aus *G*. *oxydans* DSM 4025 erhältliches Enzym B mit dem Substrat in Kontakt bringt und wobei das Enzym ausgewählt ist aus der Gruppe, bestehend aus:
(a) einer Aminosäuresequenz gemäß SEQ ID NO: 2,
(b) einer Aminosäuresequenz mit 90% Identität zu SEQ ID NO: 2 und der Aktivität zur Produktion von L-Ascorbinsäure sowie
(c) einer von einer DNA-Sequenz der SEQ ID NO: 1 oder einer mit dieser Sequenz unter stringenten Hybridisierungs- und Waschbedingungen hybridisierenden DNA-Sequenz codierten Aminosäuresequenz mit der Aktivität zur Produktion von L-Ascorbinsäure.

8. Verfahren zur Herstellung von L-Gulono-1,4-lacton oder L-Gulonsäure aus L-Gulose, wobei man in dem Verfahren aus *G*. *oxydans* DSM 4025 erhältliches Enzym B mit L-Gulose in Kontakt bringt und wobei das Enzym ausgewählt ist aus der Gruppe, bestehend aus:
(a) einer Aminosäuresequenz gemäß SEQ ID NO: 2,
(b) einer Aminosäuresequenz mit 90% Identität zu SEQ ID NO: 2 und der Aktivität zur Produktion von L-Ascorbinsäure sowie
(c) einer von einer DNA-Sequenz der SEQ ID NO: 1 oder einer mit dieser Sequenz unter stringenten Hybridisierungs- und Waschbedingungen hybridisierenden DNA-Sequenz codierten Aminosäuresequenz mit der Aktivität zur Produktion von L-Ascorbinsäure.

9. Verfahren zur Herstellung von L-Galactono-1,4-lacton oder L-Galactonsäure aus L-Galactose, wobei man in dem Verfahren aus *G*. *oxydans* DSM 4025 erhältliches Enzym B mit L-Galactose in Kontakt bringt und wobei das Enzym ausgewählt ist aus der Gruppe, bestehend aus:
(a) einer Aminosäuresequenz gemäß SEQ ID NO: 2,
(b) einer Aminosäuresequenz mit 90% Identität zu SEQ ID NO: 2 und der Aktivität zur Produktion von L-Ascorbinsäure sowie
(c) einer von einer DNA-Sequenz der SEQ ID NO: 1 oder einer mit dieser Sequenz unter stringenten Hybridisierungs- und Waschbedingungen hybridisierenden DNA-Sequenz codierten Aminosäuresequenz mit der Aktivität zur Produktion von L-Ascorbinsäure.

## Revendications

1. Utilisation d'une enzyme B pouvant être obtenue à partir de *Gluconobacter oxydans* DSM 4025 pour produire de l'acide L-ascorbique en mettant en contact ladite enzyme avec un substrat qui est choisi dans le groupe composé du L-gulose, du L-galactose, de la L-gulono-1,4-lactone, de l'acide L-gulonique, de la L-galactono-1,4-lactone et de l'acide L-galactonique ; dans laquelle ladite enzyme est choisie dans le groupe composé de :
(a) une séquence d'acides aminés selon la SEQ ID n°: 2,
(b) une séquence d'acides aminés qui est identique à 90% à la SEQ ID n°: 2, avec l'activité de production d'acide L-ascorbique, et
(c) une séquence d'acides aminés codée par une séquence d'ADN de la SEQ ID n°: 1 ou une séquence d'ADN qui s'hybride, dans des conditions stringentes d'hybridation et de lavage, avec ladite séquence ayant l'activité de production d'acide L-ascorbique.

2. Utilisation d'une enzyme B pouvant être obtenue à partir de *G*. *oxydans* DSM 4025 pour produire de la L-gulono-1,4-lactone ou de l'acide L-gulonique en mettant en contact ladite enzyme avec du L-gulose, dans laquelle ladite enzyme est choisie dans le groupe composé de :
(a) une séquence d'acides aminés selon la SEQ ID n°: 2,
(b) une séquence d'acides aminés qui est identique à 90% à la SEQ ID n°: 2, avec l'activité de production d'acide L-ascorbique, et
(c) une séquence d'acides aminés codée par une séquence d'ADN de la SEQ ID n°: 1 ou une séquence d'ADN qui s'hybride, dans des conditions stringentes d'hybridation et de lavage, avec ladite séquence ayant l'activité de production d'acide L-ascorbique.

3. Utilisation d'une enzyme B pouvant être obtenue à partir de *G*. *oxydans* DSM 4025 pour produire de la L-galactono-1,4-lactone ou de l'acide L-galactonique en mettant en contact ladite enzyme avec du L-galactose, dans laquelle ladite enzyme est choisie dans le groupe composé de :
(a) une séquence d'acides aminés selon la SEQ ID n°: 2,
(b) une séquence d'acides aminés qui est identique à 90% à la SEQ ID n°: 2, avec l'activité de production d'acide L-ascorbique, et
(c) une séquence d'acides aminés codée par une séquence d'ADN de la SEQ ID n°: 1 ou une séquence d'ADN qui s'hybride, dans des conditions stringentes d'hybridation et de lavage, avec ladite séquence ayant l'activité de production d'acide L-ascorbique.

4. Utilisation, selon l'une quelconque des revendications 1 à 3, comprenant: (a) mettre en contact l'enzyme avec le substrat respectif ; et (b) isoler le produit, qui est choisi dans le groupe composé de l'acide L-ascorbique, de la L-gulono-1,4-lactone, de l'acide L-gulonique, de la L-galactono-1,4-lactone et de l'acide L-galactonique provenant du mélange de réaction.

5. Utilisation selon l'une quelconque des revendications 1 à 4, dans laquelle le processus est conduit pendant 1 à 120 h, à un pH de 1 à 9 et à une température de 13°C à 45°C.

6. Utilisation selon la revendication 5, dans laquelle le processus est conduit à un pH de 2 à 8 et à une température de 18°C à 42°C.

7. Processus destiné à la production d'acide L-ascorbique à partir d'un substrat choisi dans le groupe composé du L-gulose, du L-galactose, de la L-gulono-1,4-lactone, de l'acide L-gulonique, de la L-galactono-1,4-lactone et de l'acide L-galactonique ; ledit processus comprenant mettre en contact une enzyme B, qui peut être obtenue à partir de *G*. *oxydans* DSM 4025, avec ledit substrat et dans laquelle ladite enzyme est choisie dans le groupe composé de :
(a) une séquence d'acides aminés selon la SEQ ID n°: 2,
(b) une séquence d'acides aminés qui est identique à 90% à la SEQ ID n°: 2, avec l'activité de production d'acide L-ascorbique, et
(c) une séquence d'acides aminés codée par une séquence d'ADN de la SEQ ID n°: 1 ou une séquence d'ADN qui s'hybride, dans des conditions stringentes d'hybridation et de lavage, avec ladite séquence ayant l'activité de production d'acide L-ascorbique.

8. Processus destiné à la production de L-gulono-1,4-lactone ou d'acide L-gulonique à partir de L-gulose, ledit processus comprenant mettre en contact une enzyme B pouvant être obtenue à partir de *G*. *oxydans* DSM 4025 avec du L-gulose et dans laquelle ladite enzyme est choisie dans le groupe composé de :
(a) une séquence d'acides aminés selon la SEQ ID n°: 2,
(b) une séquence d'acides aminés qui est identique à 90% à la SEQ ID n°: 2, avec l'activité de production d'acide L-ascorbique, et
(c) une séquence d'acides aminés codée par une séquence d'ADN de la SEQ ID n°: 1 ou une séquence d'ADN qui s'hybride, dans des conditions stringentes d'hybridation et de lavage, avec ladite séquence ayant l'activité de production d'acide L-ascorbique.

9. Processus destiné à la production de L-galactono-1,4-lactone ou d'acide L-galactonique à partir de L-galactose, ledit processus comprenant mettre en contact une enzyme B pouvant être obtenue à partir de *G*. *oxydans* DSM 4025 avec du L-gulose et dans laquelle ladite enzyme est choisie dans le groupe composé de :
(a) une séquence d'acides aminés selon la SEQ ID n°: 2,
(b) une séquence d'acides aminés qui est identique à 90% à la SEQ ID n°: 2, avec l'activité de production d'acide L-ascorbique, et
(c) une séquence d'acides aminés codée par une séquence d'ADN de la SEQ ID n°: 1 ou une séquence d'ADN qui s'hybride, dans des conditions stringentes d'hybridation et de lavage, avec ladite séquence ayant l'activité de production d'acide L-ascorbique.


### Sequence Listing

<110> Roche Vitamins AG
<120> Ascorbic Acid Production
<130> Case 21409
<150> EP 02021602
   <151> 2002-09-27
<160> 2
<170> Patent In version 3.2
<210> 1
   <211> 1740
   <212> DNA
   <213> Gluconobacter oxydans
<220>
   <221> CDS
   <222> (1)..(1740)
<400> 1
<210> 2
   <211> 579
   <212> PRT
   <213> Gluconobacter oxydans
<400> 2
   Met Asn Pro Thr Thr Leu Leu Arg Thr Ser Ala Ala Val Leu Leu Leu
   1 5 10 15
   Thr Ala Pro Ala Ala Phe Ala Gln Val Thr Pro Ile Thr Asp Glu Leu
   20 25 30
   Leu Ala Asn Pro Pro Ala Gly Glu Trp Ile Asn Tyr Gly Arg Asn Gln
   35 40 45
   Glu Asn Tyr Arg His Ser Pro Leu Thr Gln Ile Thr Ala Asp Asn Val
   50 55 60
   Gly Gln Leu Gln Leu Val Trp Ala Arg Gly Met Glu Ala Gly Ala Val
   65 70 75 80
   Gln Val Thr Pro Met Ile His Asp Gly Val Met Tyr Leu Ala Asn Pro
   85 90 95
   Gly Asp Val Ile Gln Ala Leu Asp Ala Gln Thr Gly Asp Leu Ile Trp
   100 105 110
   Glu His Arg Arg Gln Leu Pro Ala Val Ala Thr Leu Asn Ala Gln Gly
   115 120 125
   Asp Arg Lys Arg Gly Val Ala Leu Tyr Gly Thr Ser Leu Tyr Phe Ser
   130 135 140
   Ser Trp Asp Asn His Leu Ile Ala Leu Asp Met Glu Thr Gly Gln Val
   145 150 155 160
   Val Phe Asp Val Glu Arg Gly Ser Gly Glu Asp Gly Leu Thr Ser Asn
   165 170 175
   Thr Thr Gly Pro Ile Val Ala Asn Gly Val Ile Val Ala Gly Ser Thr
   180 185 190
   Cys Gln Tyr Ser Pro Tyr Gly Cys Phe Ile Ser Gly His Asp Ser Ala
   195 200 205
   Thr Gly Glu Glu Leu Trp Arg Asn His Phe Ile Pro Gln Pro Gly Glu
   210 215 220
   Glu Gly Asp Glu Thr Trp Gly Asn Asp Phe Glu Ala Arg Trp Met Thr
   225 230 235 240
   Gly Val Trp Gly Gln Ile Thr Tyr Asp Pro Val Thr Asn Leu Val Phe
   245 250 255
   Tyr Gly Ser Thr Gly Val Gly Pro Ala Ser Glu Thr Gln Arg Gly Thr
                   260                 265                 270
   Pro Gly Gly Thr Leu Tyr Gly Thr Asn Thr Arg Phe Ala Val Arg Pro
           275                 280                 285
   Asp Thr Gly Glu Ile Val Trp Arg His Gln Thr Leu Pro Arg Asp Asn
       290                 295                 300
   Trp Asp Gln Glu Cys Thr Phe Glu Met Met Val Ala Asn Val Asp Val
   305                 310                 315                 320
   Gln Pro Ser Ala Glu Met Glu Gly Leu Arg Ala Ile Asn Pro Asn Ala
                   325                 330                 335
   Ala Thr Gly Glu Arg Arg Val Leu Thr Gly Ala Pro Cys Lys Thr Gly
               340                 345                 350
   Thr Met Trp Ser Phe Asp Ala Ala Ser Gly Glu Phe Leu Trp Ala Arg
           355                 360                 365
   Asp Thr Asn Tyr Thr Asn Met Ile Ala Ser Ile Asp Glu Thr Gly Leu
       370                 375                 380
   Val Thr Val Asn Glu Asp Ala Val Leu Lys Glu Leu Asp Val Glu Tyr
   385                 390                 395                 400
   Asp Val Cys Pro Thr Phe Leu Gly Gly Arg Asp Trp Ser Ser Ala Ala
                   405                 410                 415
   Leu Asn Pro Asp Thr Gly Ile Tyr Phe Leu Pro Leu Asn Asn Ala Cys
               420                 425                 430
   Tyr Asp Ile Met Ala Val Asp Gln Glu Phe Ser Ala Leu Asp Val Tyr
           435                 440                 445
   Asn Thr Ser Ala Thr Ala Lys Leu Ala Pro Gly Phe Glu Asn Met Gly
       450                 455                 460
   Arg Ile Asp Ala Ile Asp Ile Ser Thr Gly Arg Thr Leu Trp Ser Ala
   465                 470                 475                 480
   Glu Arg Pro Ala Ala Asn Tyr Ser Pro Val Leu Ser Thr Ala Gly Gly
                   485                 490                 495
   Val Val Phe Asn Gly Gly Thr Asp Arg Tyr Phe Arg Ala Leu Ser Gln
               500                 505                 510
   Glu Thr Gly Glu Thr Leu Trp Gln Ala Arg Leu Ala Thr Val Ala Thr
   515 520 525
   Gly Gln Ala Ile Ser Tyr Glu Leu Asp Gly Val Gln Tyr Ile Ala Ile
   530 535 540
   Gly Ala Gly Gly Leu Thr Tyr Gly Thr Gln Leu Asn Ala Pro Leu Ala
   545 550 555 560
   Glu Ala Ile Asp Ser Thr Ser Val Gly Asn Ala Ile Tyr Val Phe Ala
   565 570 575
   Leu Pro Gln